# EUROPEAN PATENT SPECIFICATION

(11) **EP 1 421 100 B1**
(45) Date of publication and mention of the grant of the patent: **09.07.2008**
(21) Application number: 02772183.6
(22) Date of filing: 21.08.2002
(51) Int. Cl.: C07J 71/00

(54) **CHEMICAL SYNTHESIS OF SOLAMARGINE**
CHEMISCHE SYNTHESE VON SOLAMARGIN
SYNTHESE CHIMIQUE DE SOLAMARGINE

(30) Priority: 21.08.2001 EP 01120144
(43) Date of publication of application: 26.05.2004
(73) Proprietor: GlycoMed Sciences Limited, George Town, Grand Cayman (KY)
(72) Inventor: SHAHID, Mohammed, Reading RG6 6BZ (GB)
(74) Representative: Vossius & Partner
(86) International application number: PCT/EP2002/009349
(87) International publication number: WO 2003/018604

(56) References cited:
- US-B1- 6 214 803
- LI B ET AL: "An improved synthesis of the saponin, polyphyllin D" CARBOHYDRATE RESEARCH, ELSEVIER SCIENTIFIC PUBLISHING COMPANY. AMSTERDAM, NL, vol. 331, no. 1, 9 March 2001 (2001-03-09), pages 1-7, XP004317141 ISSN: 0008-6215
- BITE, PAL ET AL: "Preparation of steroid glycosides" ACTA CHIM. ACAD. SCI. HUNG. (1967), 52(1), 79-82 , XP001127494
- DATABASE CA [Online] CHEMICAL ABSTRACTS SERVICE, COLUMBUS, OHIO, US; BITE, PAL ET AL: "Synthesis of steroid glycosides" retrieved from STN Database accession no. 66:38204 XP002224550 & MAGY. KEM. FOLY. (1966), 72(11), 512-14 ,
- C. PACZKOWSKI ET AL: "Glucoyslation and galactosylation of diosgenin and solasodine by soluble glycosyltransferase(s) from Solanum melongena leaves" PHYTOCHEMISTRY, vol. 35, no. 6, 19 April 1994 (1994-04-19), pages 1429-1434, XP001132072

## Description

The present invention relates to the chemical synthesis of solanum glycosides, in particular to the synthesis of solamargine as well as to novel β-monosaccharide intermediate compounds.

Solasodine and its glycosides are of considerable interest clinically. They are widely used as starting products for the synthesis of various steroidal drugs. Thus the aglycon solasodine is a source for synthetic cortisone and progesterone.

It is moreover well established that certain naturally occurring conjugate solasodine glycosides have potent antineoplastic properties. Of particular interest are the triglycosides solasonine (22R, 25R)-sairo-5-en-3β-yl-α-L-rhamnopyranosyl-(1->2 gal)-O-p-D-glucopyranosyl-(1->3 gal)-β-D-galactopyranose and solamargine (22R, 25R)-spiro-5-en-3β-yl-α-L-rhamnopyranosyl-(1->2 glu)-α-L-rhamnopyranosyl- (1->4 glu)-β-D-gluco-pyranose. The structures of these triglycosides are shown below:

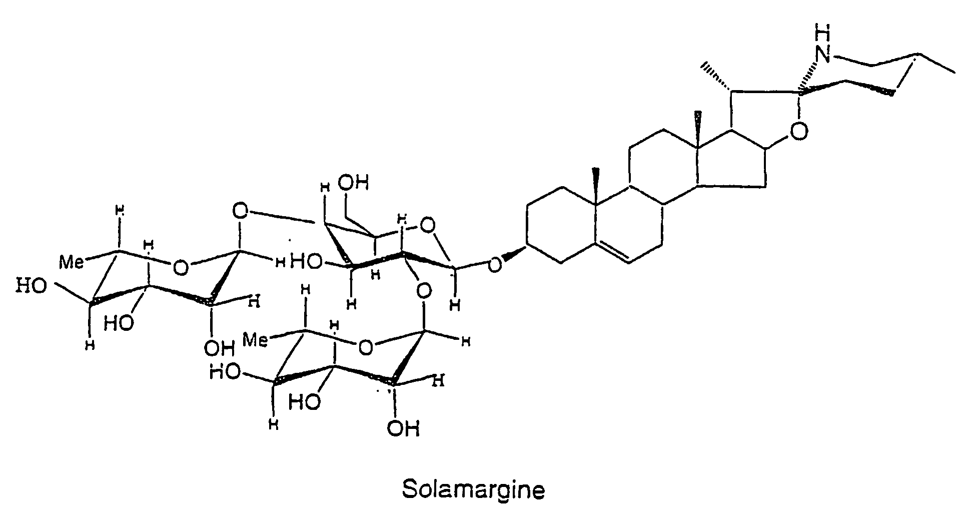

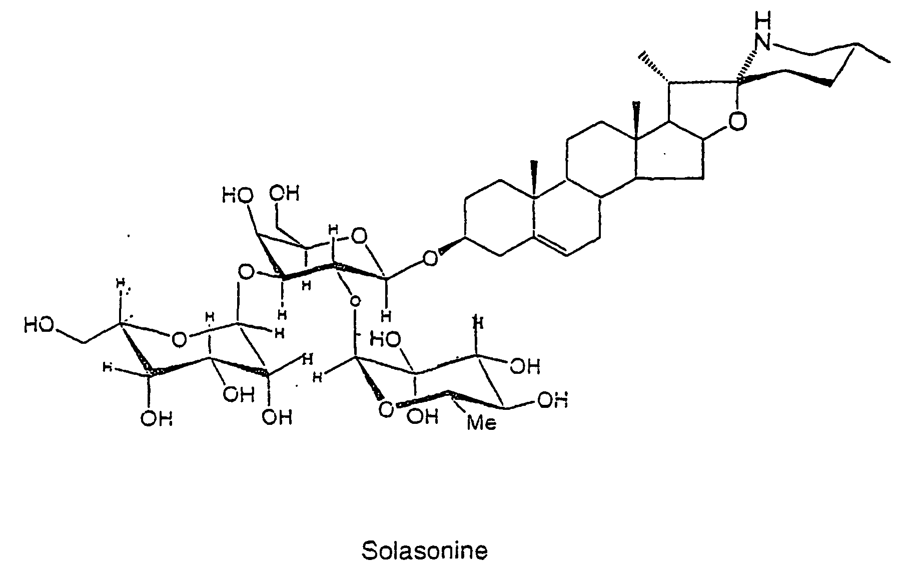

The above triglycosides are conventionally obtained by extraction from a plant source. Thus a commercially available extract of *S. sodomaeum,* commonly referred to as BEC (Drug Future, 1988, vol. 13.8, pages 714-716) is a crude mixture of solamargine, solasonine and their isomeric diglycosides. The extraction process for making BEC involves homogenizing the fruits of S. *sodomaeum* in a large volume of acetic acid, filtering off the liquid through muslin followed by precipitation of the glycosides with ammonia (Drugs of today (1990), Vol. 26 No. 1, p. 55-58, cancer letters (1991), Vol. 59, p. 183-192). The yield of the solasodine glycoside mixture is very low (approx. 1 %). Moreover the individual process steps are not defined to GMP in terms of scale up, definition of yield composition and product quality.

There is thus a great need for a cost efficient process that provides the active antineoplastic triglycoside solamargine at high yield with little or no impurities.

Contrary to other steroid ring systems, the steroid skeleton of solasodine contains a very labile nitrogen containing ring. This aglycon can thus not readily be chemically modified while keeping the steroid skeleton intact. Thus, in spite of the fact that the aglycon solasodine is readily available, the prior art does not disclose the synthesis of the solamargine using the aglycon material as starting material.

The synthesis of solamargine requires the stereoselective glycosylation of solasodine at the relatively unreactive hydroxyl group.

It has been found that solasodine is not compatible with the conventional steroid glycosylation technique. Thus no glycosylation was observed following the treatment of solasodine with tetrabenzoyl α-D-glucopyranosyl trichloroacetimidate and trimethyl-silyl triflate or trifluoride etherate (unpublished results).

The problem underlying the present invention is to provide a cost effective method for the preparation of solamargine.

The present invention resides in the finding that the stereoselective β-glycosylation of solasodine may be achieved in high yields using specific glucopyranosyl donors. Preferably the reaction is carried out in the presence of a promoter.

### Detailed description of the invention

Thus it was unexpectedly found that by reacting a D-glucopyranosyl donor of the following formula II 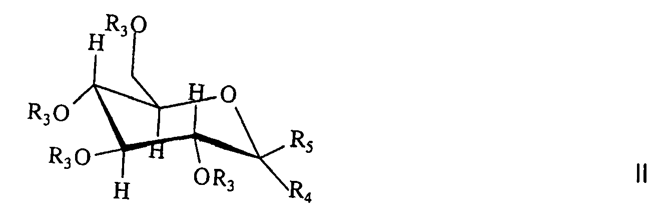 wherein each R₃ independently represents a benzoyl, acetyl or pivaloyl group, wherein R₄ is halogen selected from Cl, Br or I and R₅ is hydrogen or
R₄ is hydrogen and R₅ is SEt or SPh,
with solasodine the correspondingly protected β-glycoside of the formula I 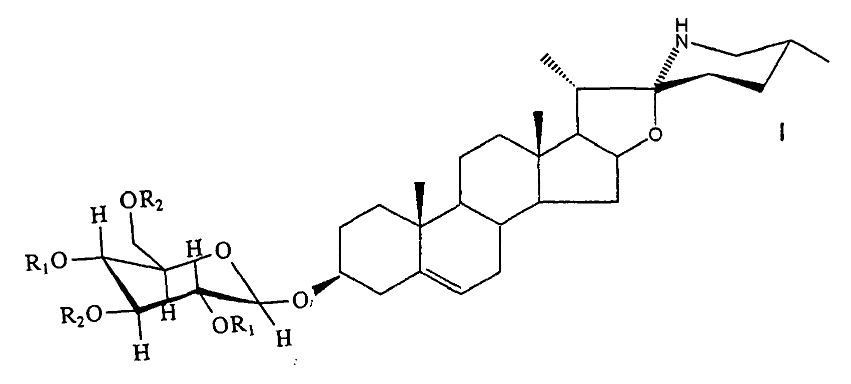 could be obtained in high yield.

As a D-glucopyranosyl donor tetra-O-benzoyl-α-D-glucopyranosyl bromide is preferred.

Preferably the reaction is carried out in the presence of a promoter.

Any conventional promoter as used in saccharide chemistry may be used.

The following promoters are particularly preferred:
Silver triflate (silver trifluoromethane sulfonate), silver trifluoromethantriflate, boron trifluoride (-10°C), diethyl etherate, trimethylsilyl triflate bromide, N-iodosuccinimide or dimethyl, thiomethyl sulfonium triflate, whereby silver triflate is most preferred.

The reaction is preferably carried out using dichloromethane as the solvent. Preferably the reaction time is 30 min.-1 hr at -20°C.

The β-glycosides of formula I may be used as intermediates for the synthesis of solamargine: 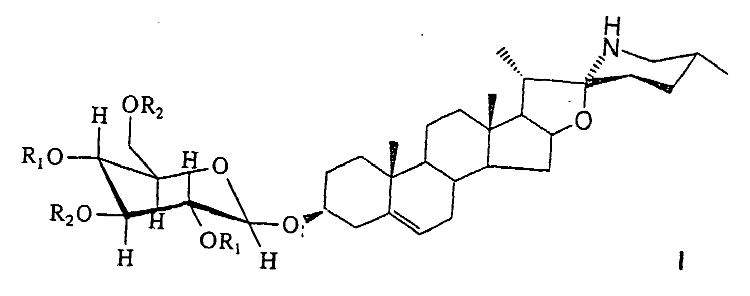 wherein each of R1 and R2, which may be the same or different, represent a benzoyl, pivaloyl or acetyl.

The desired end product solamargine may be prepared by deprotecting the β-glycoside of formula I to obtain a compound of the formula V 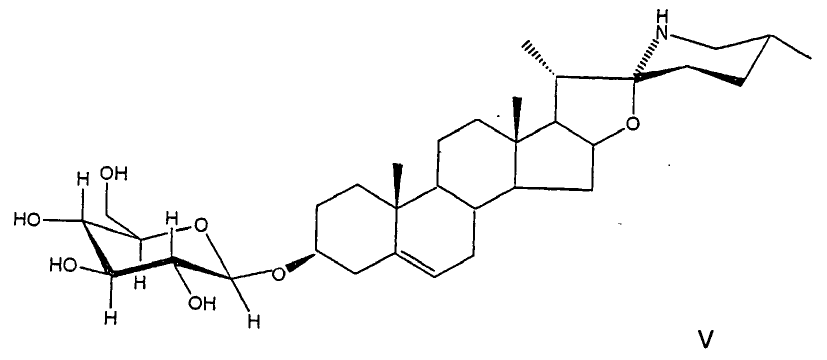 and reesterifying the most reactive hydroxyl groups (OH-3 and OH-6) using conventional protecting groups to obtain a compound of formula IIa, wherein R2 is as defined above, whereby pivaloyl is preferred as a protection group,

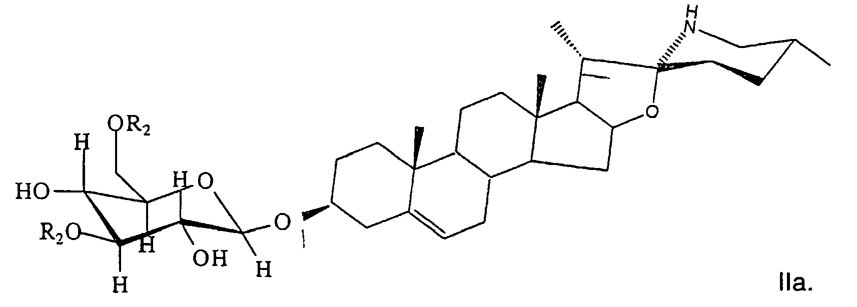

The partially protected β-glycoside diol is then glycosylated at OH-2 and OH-4 with a suitable α-L-rhamnopyranosyl donor.

Suitable rhamnose donors include tri-O-benzoyl-α-rhamnopyranosyl bromide, tri-O-pivaloyl-α-L-rhamnopyranosyl trichloroacetoimidate or a glycoside of formula IV 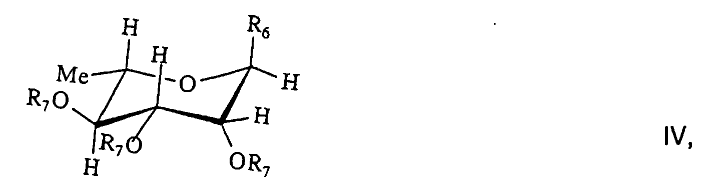 wherein R₆ is Br, CI, I, SEt or SPh and

R₇ is benzoyl, acetyl or pivaloyl. Tri-O- benzoyl-α- L-rhamnopyranosyl bromide is preferred as the rhamnose donor.

The protected solamargine of formula III (1) 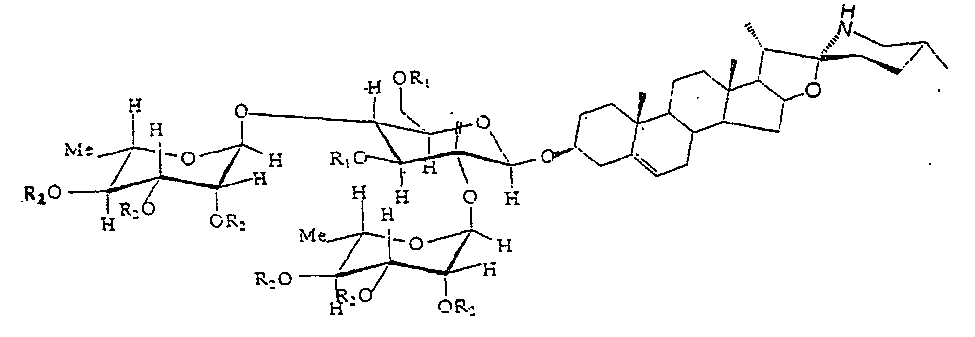
(1) R₁ = benzoyl, acetyl or pivaloyl
   R₂ = " "
(2) R₁ = R₂ = H
may be deesterified to produce compound III(2) in a conventional manner, e.g. by treating the protected solamargine with a base such as sodium methoxide or sodium hydroxide in a methanol-dichloromethane solution or a methanol-tetrahydrofuran-water mixture, followed by neutralization with e.g. solid CO₂ or mild acid ion-exchange resin such as Amberlyst® 50H⁺ or Dowex® (H⁺ form). These ion -exchange resins may also be used in any other deprotection step in the synthesis according to the invention.

The Examples described below serve only illustrative purposes and are not construed to limit the scope of the invention.

### Synthesis Example 1

### Step A Preparation of bromo 2,3,4,6,-tetra-O-benzoyt-α-D-glucopyranose (tetra-O-benzoyl-α-D-glucopyranosyl bromide)

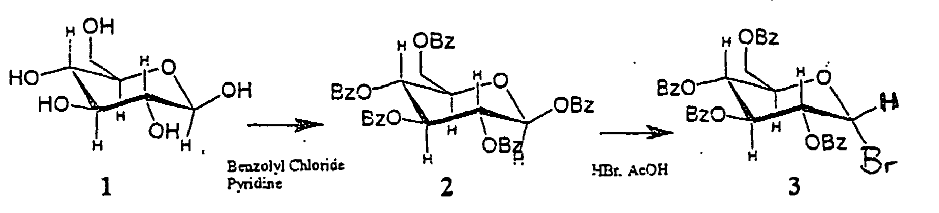

D-Glucose (30g) (1) was placed in a 1 liter three necked round bottomed flask equipped with stirrer and thermometer. Pyridine (300ml) was added and the mixture gently heated to aid dissolution. The mixture was cooled to 10-12°C using ice/water and benzoyl cloride (116ml) added dropwise over a period of 40 min (temperature reached 20°C). After about 90ml of the benzoyl chloride had been added the mixture became more viscous and a light yellow precipitate formed. After the benzoyl chloride addition was complete, the mixture was left stirring overnight at room temperature when a light brown slurry formed. Water (400ml) was added and the mixture extracted with dichloromethane (DCM) (3x 800ml). The organic phase was separated and washed with water (600ml), 1 N HCl (2x 600ml) and saturated NaHCO₃ (800ml). The organic phase was dried (MgSO₄), filtered and the solvent removed to leave a thick oil (2).

The fully benzoylated glucose (2) was then dissolved in dichloromethane (200ml) and cooled to 0°C in a ice/water bath. Hydrogen bromide (32% in acetic acid, (142ml)) was then added dropwise over 30 min to the reaction mixture. When addition was complete, the mixture was allowed to reach room temperature and stirred overnight. Dichloromethane (400ml) (DCM) was added and the mixture washed with ice-water (4 x 500ml), saturated NaHCO₃ (3x 500ml), dried with MgSO₄ and filtered through activated charcoal. The solvent was removed under reduced pressure to leave a light yellow oil which solidified on standing. The title compound (3) was recrystallised from diethylether (800ml) and petroleum ether (700ml) to give 85 g as an off white solid.

### Step B Preparation of Bromo 2,3,4-tri-O-benzoyl-rhamnopyranose (tri-O-benzoyl-α-rhamnopyranosyl bromide)

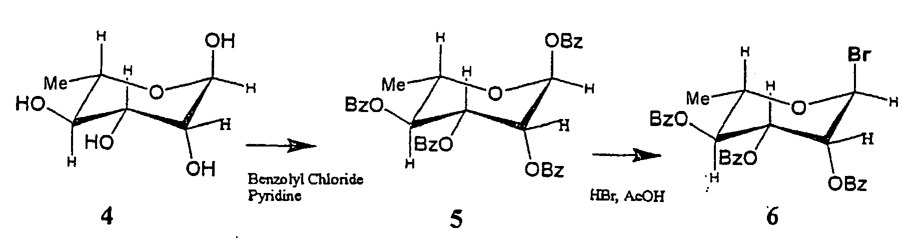

L-Rhamnose (20g) (4) was placed in a 250ml round bottomed flask equipped with stirrer, thermometer and pressure equalising dropping funnel. Pyridine (25ml) was added and the mixture cooled to 0°C using a ice/water bath. Benzoyl chloride (90ml) was then added dropwise over 20min, and after the addition was complete the mixture was heated at 60°C for 2h. After the mixture had cooled to room temperature, water (30ml) was added, stirred for 20min and then diluted with dichloromethane (DCM) (500ml). The mixture was washed with cold water (2x200ml), 1 N HCl (3x25ml), saturated NaHCO₃ (306ml) and saturated brine (300ml). The organic phase was dried over MgSO₄, filtered through activated charcoal and the solvent removed under reduced pressure to give a thick syrup (5).

The fully benzoylated rhamnose (5) was dissolved in acetic acid (30ml) and the solution cooled to 0°C. Hydrogen bromide (32% in acetic acid) was then added dropwise over 20min. When addition was complete, the mixture was allowed to reach room temperature and was stirred overnight (18h).

Dichloromethane (400ml) was added and the mixture was washed with ice/water (2x 200ml), dried over MgSO₄, filtered and the solvent removed under reduced pressure to give an oil that crystallized on standing. Recrystallisation from toluene/petroleum ether provided the title compound (6) 23g as a white solid.

### Step C Preparation of solasodine-2,3,4,6-tetra-O-benzoyl-glucose (tetra-O-benzoyl-sotasod-5en-3β-yl-D-glucopyranoslde)

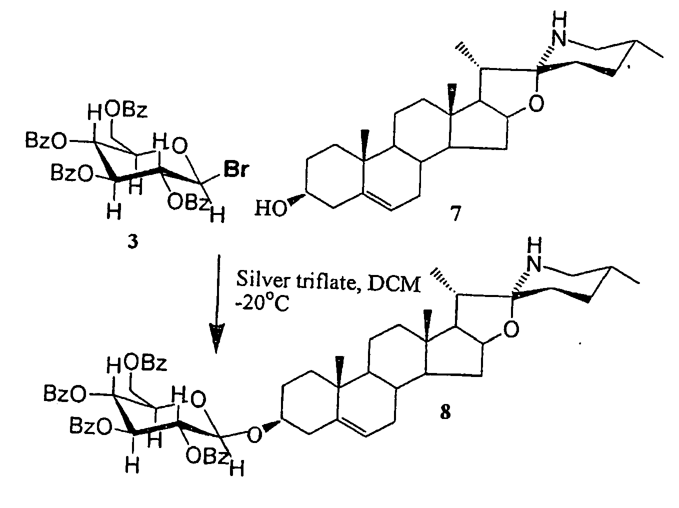

Solasodine (7) (15g, 302mmol), bromo-benzoyl-glucose (tetra-O-benzoyl-α-D-glucopyranosyl bromide) (27g, 544mmol) and 4Å molecular sieve (crushed to a powder and preheated in a vacuum oven at 60°C), were placed in a 500ml round bottom flask equipped with nitrogen inlet-bubbler, pressure equalizing dropping funnel and low temperature thermometer. Anhydrous dichloromethane (250ml) was then added and the mixture was stirred at room temperature under argon for 40 min. The mixture was then cooled to -20°C. A solution of silver trifluoromethane sulfonate (14g, 544mmol) in anhydrous toluene was then added dropwise over 15 min to the cold reaction mixture. After addition, the mixture was slowly allowed to warm to 5°C yielding a viscous pale precipitate. Thin layer chromatography (TLC) of the reaction mixture (eluent; 10:90 MeOH: DCM) with solasodine (7) R_{f}=0.42, showed a spot at R_{f}=0.5 and excess bromo-glucose (3) at R_{f}=0.8. The reaction mixture was filtered through a pad of celite and washed with DCM (200ml). The filtrate was then concentrated under reduced pressure to leave a thick dark brown oil. Silica gel chromatography (eluent; 40:60; EtOAc: toluene to 50:50 EtOAc: toluene) provided the pure product (8) (33.4g,100%) as a light orange crusty solid.

### Step D Benzoyl deprotection

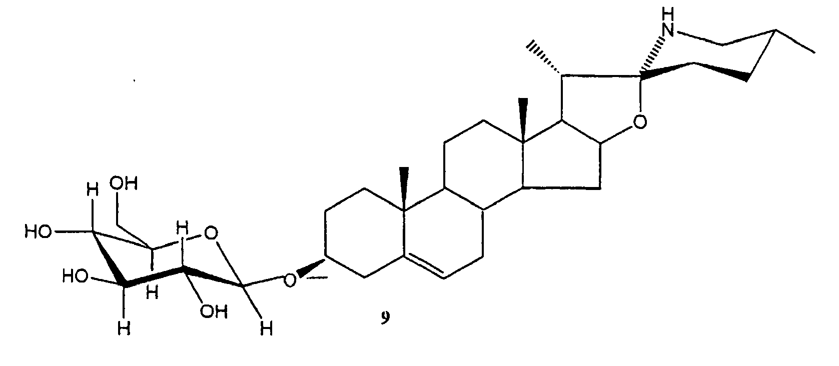

The fully protected solasodine-glucose adduct (8) (37g, 377mmol) was dissolved in methanol (400 ml) and dichloromethane (200ml).To the homogenous mixture sodium methoxide (12 ml, 25 % by weight in methanol) was added and the reaction was mixture stirred at room temperature for 2 h. Thin layer chromatography (eluent: 10:90; MeOH:DCM) of the reaction mixture showed no remaining protected adduct (R_{f}=0.5, UV active) and only a higher running spot at R_{f}= 0.9 (methylbenzoate), the product appeared at R_{f}= 0.15 (UV inactive, visualized by H₂SO₄/MeOH charring). Amberlyst® 15H⁺ resin was activated by washing in 1 N HCl, filtered and then added to the reaction mixture until the pH was between 7 and 8. The solvent was then removed under reduced pressure to give a thick brown syrup. Silica gel chromatography (eluent: 10:90; MeOH:DCM to 20:88:2: MeOH:DCM:NH₃ aq.) provided the pure deprotected product (9) (16.4g, 76%).

Thin layer chromatography (20:88:2; MeOH:DCM:NH₃aq.) showed the product (at R_{f}=0.55 (UV inactive, with H₂SO₄/MeOH charring)

### Step E Selective pivaloylation (3,6) of the glucose-solasodine adduct

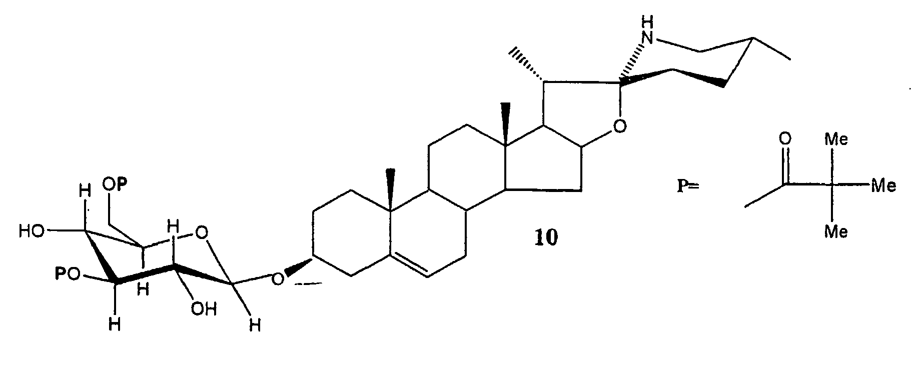

The glucose-solasodine adduct of step D (16 g, 278mmol) (9) and pyridine (120ml) were placed in a three necked flask equipped with a stirrer and a thermometer. The solution was cooled to 0°C using ice/water and pivaloyl chloride (6.7g, 556 mmol) was added dropwise over a period of 20 min. After about 30 min a TLC sample was divided between water and ethylacetate; the ethylacetate layer was used (eluent: 5:95; MeOH:DCM), which showed only the monopivaloyl product at R_{f}=0.2 and some of the starting material at R_{f}=0.1. Another 2 equivalents of pivaloyl chloride (6.7g) was added (TLC as before showed no starting material and only mono- and dipivaloyl products, at R_{f}=0.2 and 0.4, respectively). After another 90 min at 0°C TLC showed only the dipivaloyl product and some minor impurities. The reaction mixture was diluted with ethylacetate and washed 3x with 5% HCl aq. (250ml). The organic phase was washed with saturated brine, dried (MgSO₄), filtered and the solvent removed under reduced pressure to yield a brown oil. Silica gel chromatography (eluent: 5:95; MeOH:DCM to 10:90 MeOH:DCM) provided the product (10) as an off white solid (15g, 72%).

### Step F Addition of two moles of rhamnose to the 3,6-protected glucose-solasodine adduct

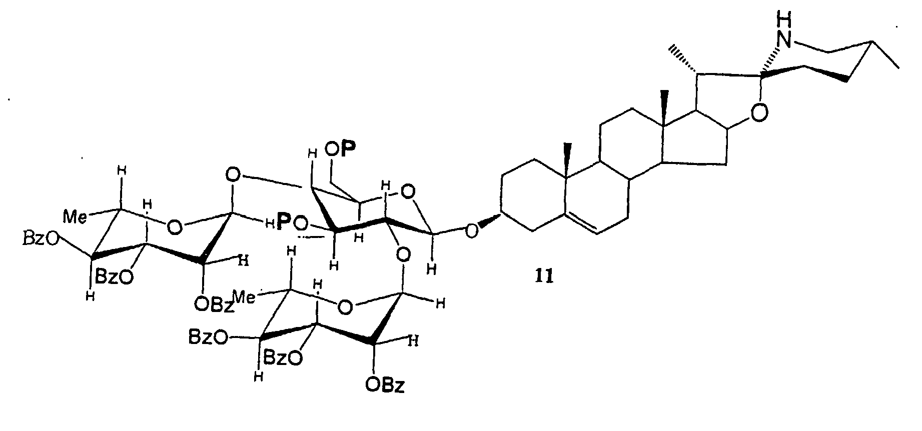

The rhamnose bromide from step B (6)(19g, 355mmol), dipivaloyl-glucose-solasodine (12g, 161mmol), 4Å molecular sieves (30g, preheated in an oven at 50°C) were placed in a three necked flask equipped with a stirrer, a low temperature thermometer, an argon inlet-bubbler and a pressure dropping equalizing funnel. Anhydrous DCM was added and the mixture was stirred at room temperature for 40 min under argon.

The mixture was then cooled to -30°C and a solution of silver triflate in toluene (80ml) was added dropwise over 20 min maintaining the temperature at -30°C. After addition the resulting light yellow precipitate was stirred at -20°C for 30 min and then slowly allowed to reach -5°C over 35 min. A TLC sample (eluent: 5:95; MeOH:DCM) showed a UV active product at R_{f}=0.4 and no starting material at R_{f}=0.3. 3ml triethylamine was added and the mixture was diluted with DCM and filtered through a pad of celite. The celite was washed with DCM (200ml) and the filtrate was evaporated under reduced pressure to leave a purple coloured syrup. Silica gel chromatography (5:95 to 10:90; MeOH:DCM) provided the pure fully protected solamargine (11) (13,6g, 50%) as a crustly light yellow solid.

### Step G Deprotection of fully protected solamargine

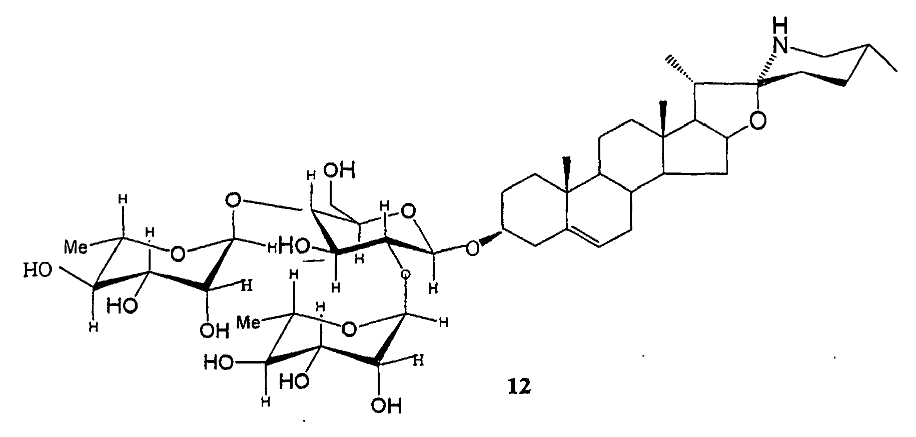

A mixture of THF, MeOH and water (1:1:1; 20:20:20 ml) was added to the fully protected solamargine (11) (8g, 4.8mmol). Sodium hydroxide (1.9g, 48 mmol) was then added and the resulting reaction mixture was heated gently at 40°C for 18 h. The mixture was cooled and diluted with methanol (50ml) and neutralized with Amberlyst 50H⁺ until the pH was between 7 and 8. The mixture was filtered and the filtrate was evaporated under reduced pressure to give a thick brown semisolid mass. TLC showed a product at R_{f}=0.45 (eluent: 70:28:2; DCM:MeOH:NH₃ aq.), which was identical to authentic solamargine.

Silica gel chromatography (eluent: 80:20; DCM:MeOH to 78:20:2 DCM:MeOH:NH₃aq) gave a product (3.4g) at R_{f}=0.45 with a close running spot at R_{f}=0.47.

### Further purification:

The semi solid paste was taken up in 3% acetic acid (450ml) and stirred for 20 min until nearly all dissolved. The solution was decanted from any undissolved material and adjusted with concentrated ammonia to pH 8 at which point a fine precipitate formed. The precipitate was subjected to high speed centrifugation and the supernatant decanted from the pellet that had formed. The pellet was then washed three times with water and then dispersed in water (100ml) and freeze dried to give a light white solid (1.52g).The 78% pure solamargine (12) was then further purified with conventional reversed phase HPLC chromatography.

The obtained product solamargine [(22R, 25R)-spiro-5-en-3β-yl-α-L-rhamnopyranosyl-(1-2glu)-O-α-L-rhamnopyranosyl-(1-4glu)-β-D-glucopyranose] was subjected further analysis.

A sample of the final compound was analysed by positive ion Electrospray -Mass Spectroscopy using a PE Sciex API 150 EX single quadrupole mass spectrometer. A major signal was observed at m/z 868.7 which is consistent with the expected protonated monoisotopic mass of solamargine.

Moreover the HPLC retention time of synthetic Solamargine was identical to authentic natural material, as was the thin layer chromatography (TLC).

The NMR and mass spectra were consistent with the expected structure.

### Synthesis Example 2

### Step C Preparation of solasodine-2,3,4,6-tetra-O-benzoyl-glucose (tetra-O-benzoyl-solasod-5en-3β-yl-D-glucopyranoside)

Solasodine (7) (3.6g from Research Plus Inc, USA) and tetra-O-benzoyl-α-D-glucopyranosyl bromide (3) (8.60g) in dichloromethane (120ml) was stirred with powdered molecular sieve (4Å) for 50 min. with cooling to -12°C. A solution of silver triflate (3.35g) in toluene (30ml) was added dropwise over 20 min at -12 to -10°C. The mixture was stirred for a further 30 min with slow warming to -5°C. The mixture was then filtered through Celite and washed with dichloromethane. The filtrate was washed with brine, saturated aqueous sodium bicarbonate (twice), again brine, dried, filtered and concentrated onto silica gel. The material was then chromatographed on silica eluting with EtOAc: toluene (40:60) and the combined fractions concentrated to a white solid (8). The yield was 7.13g and the sample was crystallised by trituration with ethanol. The NMR and mass spectra were consistent with the expected structure.

### Step D Benzoyl deprotection

A solution of tetra-O-benzyl-solasod-5en-3β-yl-β-D-glucopyranoside (8) (1.0g) and sodium methoxide (0.2g) in dichloromethane (80ml), and methanol was stirred overnight at 50°C. The resulting mixture was allowed to cool and neutralized with Dowex-50 (H⁺ form), filtered and the solvent removed under reduced pressure to leave a solid residue which was finally purified by silica chromatography to obtain the pure deprotected material (9).

The NMR and mass spectra were consistent with the expected structure.

## Claims

1. A method for the preparation of the glucose-solasodine conjugate of formula IIa comprising the reaction of solasodine with a glucopyranosyl donor of general formula II 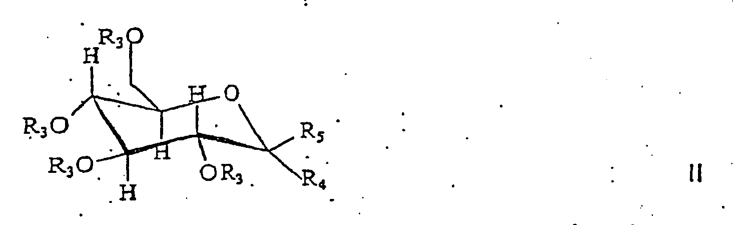 wherein each R₃ independently represents a benzoyl, acetyl or pivaloyl group,
wherein R₄ is halogen selected from Cl, Br or I and R₅ is hydrogen or
R₄ is hydrogen and R₅ is SEt or SPh,
followed by de-protecting the obtained glycoside to yield a compound of the formula V 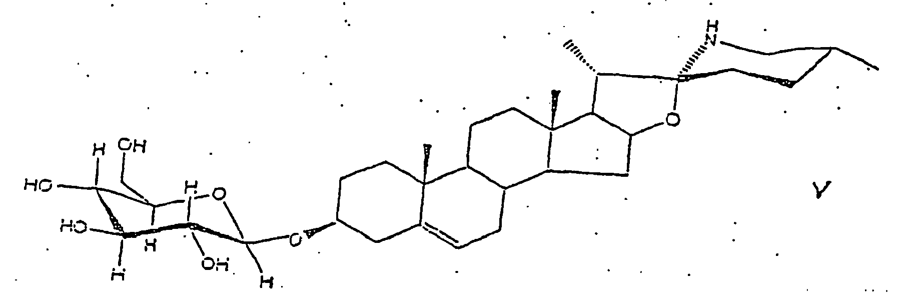 and reesteritication ot the most reactive hydroxyl groups OH-3 and OH-6 to yield a compound of the formula IIa 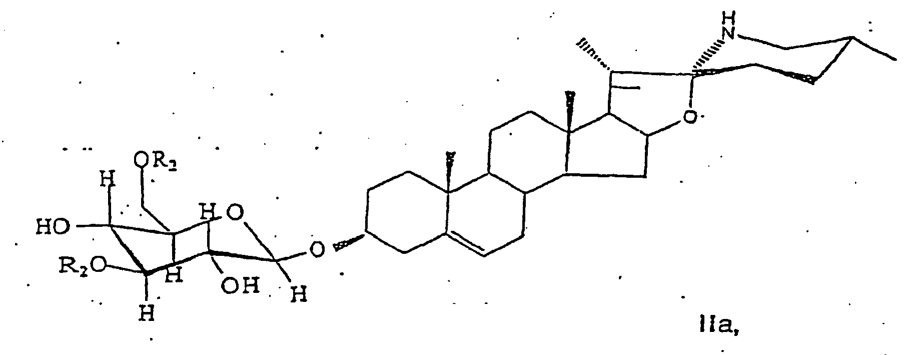 wherein R₂ is a group selected from benzoyl, pivaloyl or acetyl.

2. A method for the preparation of solamargine comprising the glycosylation of the diol of formula IIa 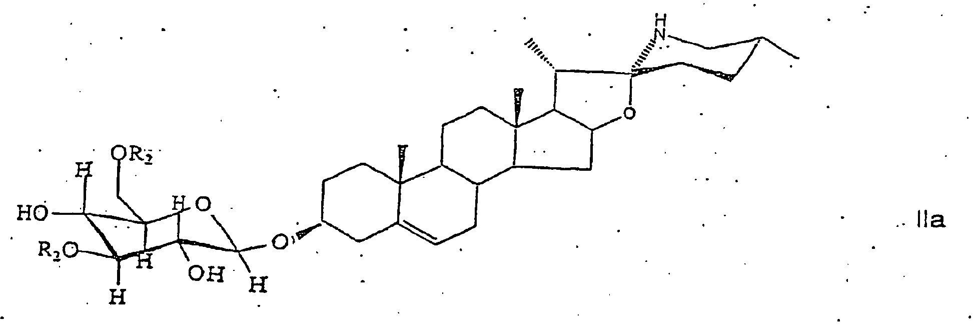 wherein R₂ is defined as in claim 1 with an α-L-rhamnopyranosyl donor to yield protected solamargine of formula III (1) which is de-esterified to yield solamargine of formula III (2) 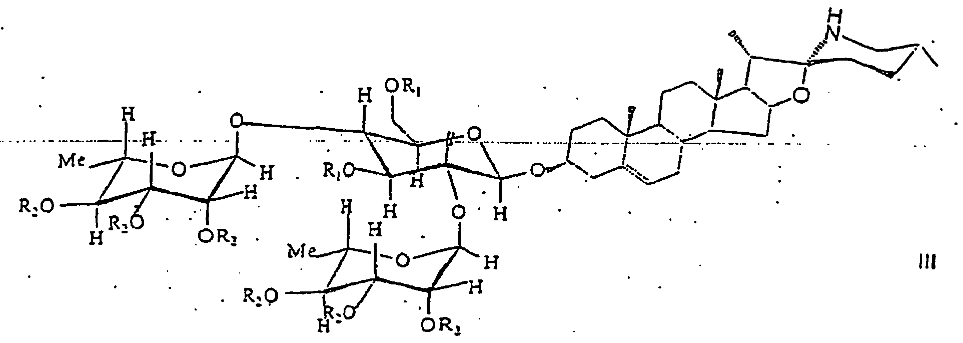
(1) R₁=Benzoyl, acetyl or pivaloyl and R₂=Benzoyl, Acetyl or pivaloyl
(2) R₁=R₂=H

3. The method according to claim 1, wherein the D-glucosepyranosyl donor is tetra-O-benzoyl-α-D-glucopyranosyl bromide, tetra-O-acetyl-α-D-glucopyranosyl bromide or tetra-O-pivaloyl-α-D-glucopyranosyl bromide.

4. The method according to claim 1 or 3, wherein the glycosylation reaction is carried out in the presence of a promoter selected from silver trifluoromethane sulfonate boron trifluoride diethyl etherate, trimethylsilyl triflate bromide, N-jodosuccinimide or dimethyl thiomethyl sulfonium triflate, silver trifluoromethyltriflate.

5. The method of claim 1, wherein the protected glycoside is deprotected in methanol-dichloromethane solution by treatment with sodium methoxide, followed by neutralization with solid CO₂ or mild acid ion-exchange resin.

6. The method of claim 1, wherein the most reactive hydroxyl groups OH-3 and OH-6 are protected by reesterification with pivaloyl chloride in pyridine solution.

7. The method of claim 2, wherein the rhamnose donor is tri-O-benzoyl-α-L-rhamnopyranosyl bromide, tri-O-pivaloyl-α-L-rhamnopyranosyl trichloroacetimidate or a glycoside of the general formula IV 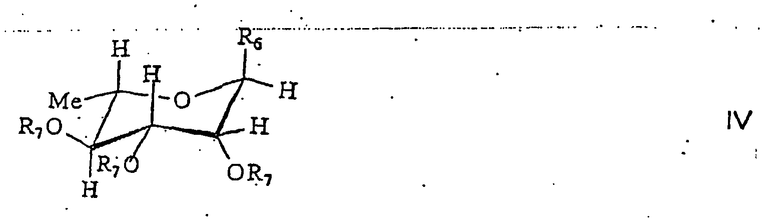 wherein R₆ is Br, Cl, I, SEt or SPh and
R₇ is benzoyl, acetyl or pivaloyl.

8. The method of claim 2, wherein the protected solamargine is de-esterified by treatment with a base selected from sodium methoxide or sodium hydroxide in methanol-dichloromethane solution or a methanol-tetrahydrofuran-water mixture followed by neutralization with solid CO₂ or mild acid ion-exchange resin.

## Patentansprüche

1. Verfahren zur Herstellung von Glucose-Solasodin-Konjugat der Formel IIa, das die Umsetzung von Solasodin mit einem Glucopyranosyldonor der allgemeinen Formel II 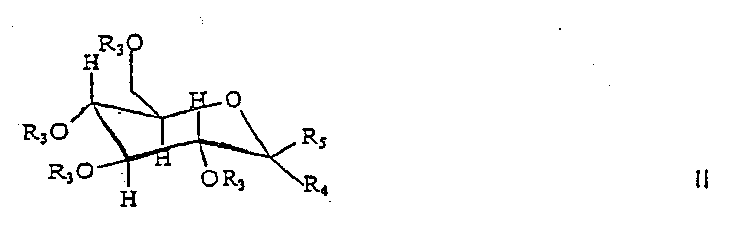 umfasst,
wobei jedes R₃ unabhängig für einen Benzoyl-, Acetyl- oder Pivaloylrest steht,
wobei R₄ ein Halogenatom ausgewählt aus Cl, Br oder I ist und R₅ ein Wasserstoffatom ist oder
R₄ ein Wasserstoffatom ist und R₅ SEt oder SPh ist,
gefolgt vom Entfernen der Schutzgruppen vom erhaltenen Glycosid, um eine Verbindung der Formel V zu ergeben 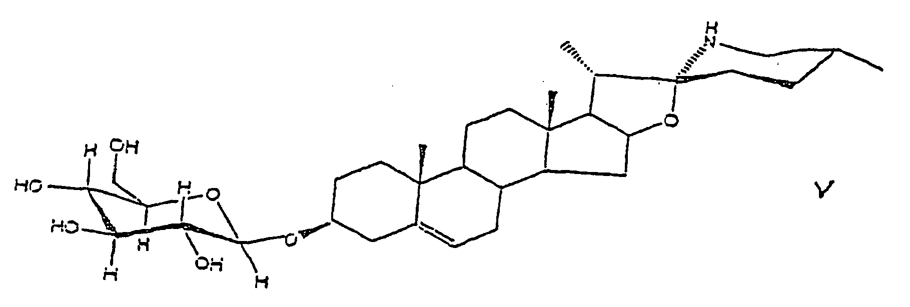 und Wiederveresterung der reaktivsten Hydroxylgruppen OH-3 und OH-6, um eine Verbindung der Formel IIa zu ergeben 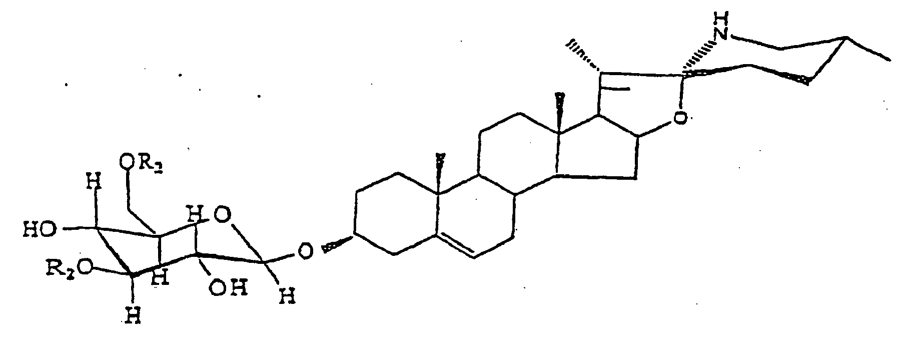 wobei R₂ ein Rest ausgewählt aus Benzoyl, Pivaloyl oder Acetyl ist.

2. Verfahren zur Herstellung von Solamargin, das die Glycosylierung des Diols der Formel IIa 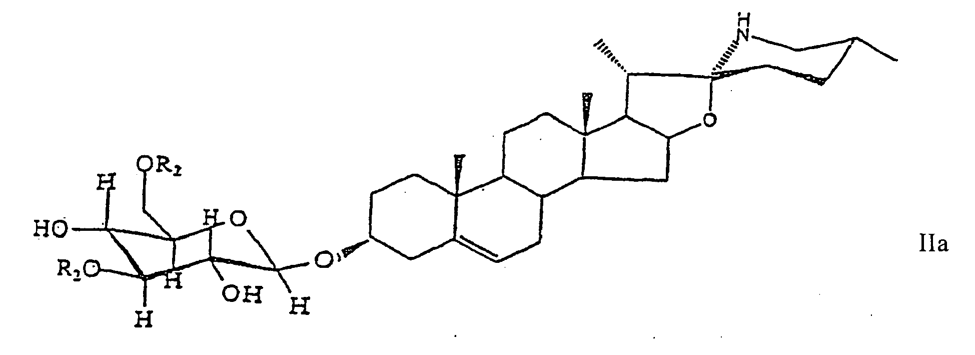 wobei R₂ wie in Anspruch 1 definiert ist, mit einem α-L-Rhamnopyranosyldonor umfasst, um geschütztes Solamargin der Formel III (1) zu ergeben, bei dem die Estergruppen abgespalten werden, um Solamargin der Formel III (2) zu ergeben 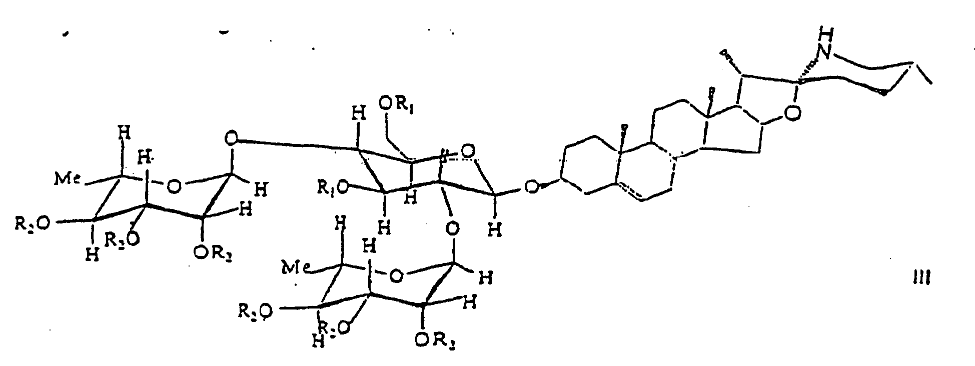
(1) R₁ = Benzoyl, Acetyl oder Pivaloyl und R₂ = Benzoyl, Acetyl oder Pivaloyl
(2)R₁=R₂=H

3. Verfahren gemäß Anspruch 1, wobei der D-Glucosepyranosyldonor Tetra-O-benzoyl-α-D-glucopyranosylbromid, Tetra-O-acetyl-α-D-glucopyranosylbromid oder Tetra-O-pivaloyl-α-D-glucopyranosylbromid ist.

4. Verfahren gemäß Anspruch 1 oder 3, wobei die Glycosylierungsreaktion in der Gegenwart eines Promotors ausgewählt aus Silbertrifluormethansulfonat, Bortrifluoriddiethyletherat, Bromtrimethylsilyltriflat, N-Iodsuccinimidtriflat oder Dimethylthiomethylsulfoniumtriflat, Silbertrifluormethyltriflat durchgeführt wird.

5. Verfahren gemäß Anspruch 1, wobei vom geschützten Glycosid in Methanol-Dichlormethan-Lösung durch Behandlung mit Natriummethoxid die Schutzgruppen abgespalten werden, gefolgt von Neutralisierung mit festem CO₂ oder schwach saurem Ionenaustauscherharz.

6. Verfahren gemäß Anspruch 1, wobei die reaktivsten Hydroxylgruppen OH-3 und OH-6 durch Wiederveresterung mit Pivaloylchlorid in Pyridinlösung geschützt werden.

7. Verfahren gemäß Anspruch 2, wobei der Rhamnosedonor Tri-O-benzoyl-α-L-rhamnopyranosylbromid, Tri-O-pivaloyl-α-L-rhamnopyranosyltrichloracetimidat oder ein Glycosid der allgemeinen Formel IV 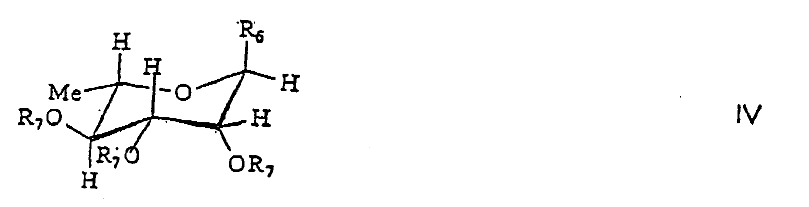 ist,
wobei R₆ Br, Cl, I, SEt oder SPh ist und
R₇ Benzoyl, Acetyl oder Pivaloyl ist.

8. Verfahren gemäß Anspruch 2, wobei vom geschützten Solamargin die Estergruppen durch Behandlung mit einer Base ausgewählt aus Natriummethoxid oder Natriumhydroxid in Methanol-Dichlormethan-Lösung oder einem Methanol-Tetrahydrofuran-Wassergemisch abgespalten werden, gefolgt von Neutralisierung mit festem CO₂ oder schwach saurem Ionenaustauscherharz.

## Revendications

1. Méthode de préparation d'un conjugué glucose-solasodine de formule IIa comprenant la réaction de la solasodine avec un donneur de glucopyranosyle de formule générale II 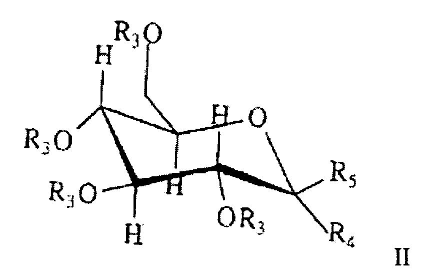 dans laquelle chacun des R₃ représente indépendamment un groupe benzoyle, acétyle ou pivaloyle,
dans laquelle R₄ est un halogène sélectionné parmi Cl, Br ou I et R₅ est un hydrogène ou R₄ est un hydrogène et R₅ est SEt ou SPh,
suivie par une déprotection du glucoside obtenu pour donner un composé de formule V 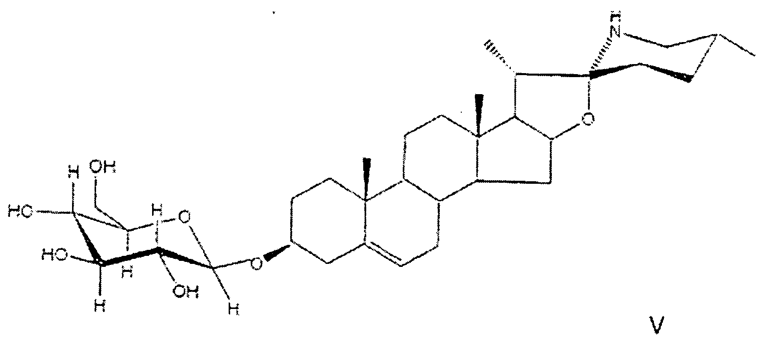 et la réesterification des groupes hydroxyles les plus réactifs OH-3 et OH-6 pour donner un composé de formule IIa 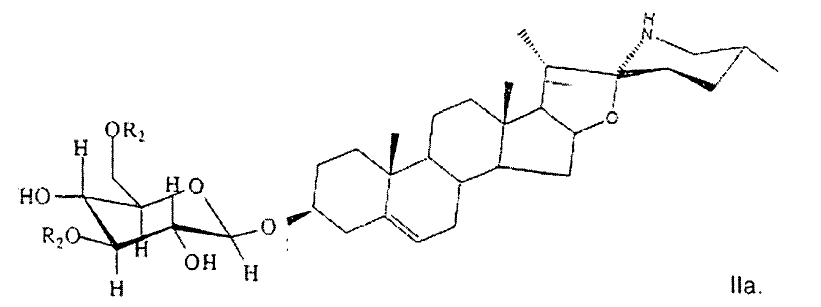 dans laquelle R₂ est un groupe sélectionné parmi un benzoyle, un pivaloyle ou un actéyle.

2. Méthode de préparation de la solamargine comprenant la glycosylation du diol de la formule IIa 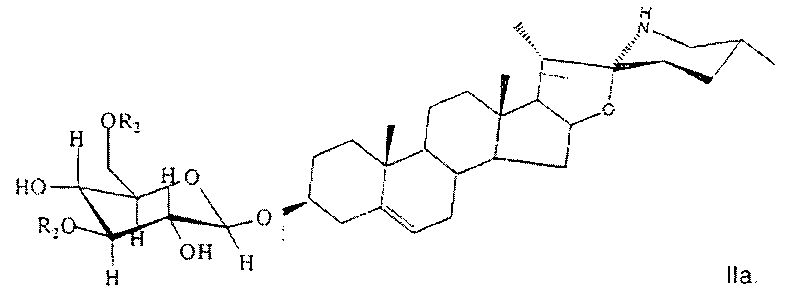 dans laquelle R₂ est tel que défini dans la revendication 1 avec un donneur de α-L-rhamnopyranosyle
pour donner une solamargine protégée de formule III (1) qui est dés-estérifiée pour donner la solamargine de formule III (2) 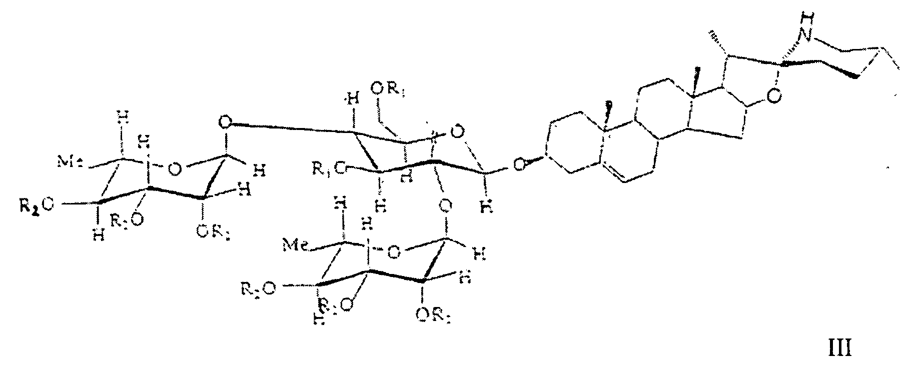
(1) R₁ = benzoyle, acétyle ou pivaloyle et R₂ = benzoyle, acétyle ou pivaloyle
(2) R₁ = R₂ = H

3. Méthode selon la revendication 1, dans laquelle le donneur de D-Glucose pyranosyle est le bromure de tétra-O-benzoyl-α-D-glucopyranosyle, le bromure de tétra-O-acétyl-α-D-glucopyranosyle ou le bromure de tétra-O-pivaloyl-α-D-glucopyranosyle.

4. Méthode selon la revendication 1 ou 3, dans laquelle la réaction de glycosylation est effectuée en présence d'un promoteur sélectionné parmi le trifluorométhane sulfonate d'argent, le trifluoroborane diéthyl éthérate, le bromure de triméthylsilyl triflate, le triflate de N-iodosuccinimide ou de diméthyl thiométhyl sulfonium, et le trifluorométhyl triflate d'argent.

5. Méthode selon la revendication 1, dans laquelle le glucoside protégé est déprotégé dans une solution de méthanol-dichlorométhane par traitement avec du méthoxide de sodium, suivi par une neutralisation avec du CO₂ solide ou une résine échangeuse d'ion légèrement acide.

6. Méthode selon la revendication 1, dans laquelle les groupes hydroxyles les plus réactifs OH-3 et OH-6 sont protégés par ré-estérification avec du chlorure de pivaloyle dans une solution de pyridine.

7. Méthode selon la revendication 2, dans laquelle le donneur de rhamnose est le bromure de tri-O-benzoyl-α-L-rhamnopyranosyle, le trichloroacétimidate de tri-O-pivaloyl-α-L-rhamnopyranosyle, ou un glucoside de formule générale IV 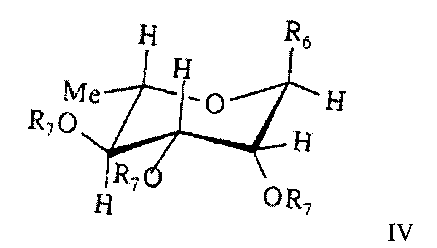 dans laquelle R₆ est Br, Cl, I, SEt ou SPh et
R₇ est un benzoyle, un actéyle ou un pivaloyle.

8. Méthode selon la revendication 2, dans laquelle la solamargine protégée est dés-estérifiée par traitement avec une base sélectionnée parmi le méthoxide de sodium ou l'hydroxyde de sodium dans une solution de méthanol-dichlorométhane ou un mélange de méthanol-tétrahydrofurane-eau suivie par une neutralisation avec du CO₂ solide ou une résine échangeuse d'ion légèrement acide.
